# EUROPEAN PATENT SPECIFICATION

(11) **EP 3 260 095 B1**
(45) Date of publication and mention of the grant of the patent: **17.10.2018**
(21) Application number: 15882746.9
(22) Date of filing: 18.12.2015
(51) Int. Cl.: A61F 13/15, A61F 13/49

(54) **METHOD AND APPARATUS FOR PRODUCING CONTINUOUS SHEET COMPOSITES FOR ABSORPTION ARTICLES**
VERFAHREN UND VORRICHTUNG ZUR HERSTELLUNG VON ENDLOSBAHNVERBUNDSTOFFEN FÜR ABSORBIERENDE ARTIKEL
PROCÉDÉ ET APPAREIL DE PRODUCTION DE COMPOSITES STRATIFORMES CONTINUS POUR ARTICLES D'ABSORPTION

(30) Priority: 19.02.2015 JP 2015030207
(43) Date of publication of application: 27.12.2017
(73) Proprietor: Unicharm Corporation, Shikokuchuo-shi, Ehime 799-0111 (JP)
(72) Inventor: NAKANO, Takumi, Kanonji-shi Kagawa 769-1602 (JP)
(74) Representative: Staeger & Sperling Partnerschaftsgesellschaft mbB
(86) International application number: PCT/JP2015/085477
(87) International publication number: WO 2016/132645

(56) References cited:
- WO-A1-2014/185242
- JP-A- S60 214 959
- JP-A- S61 162 462
- JP-A- 2006 340 902
- JP-A- 2007 117 226
- US-A1- 2003 051 802
- US-A1- 2003 121 614

## Description

### Technical Field

The present invention relates to a manufacturing method and manufacturing apparatus for a composite body of a continuous-sheet associated with an absorbent article such as disposable diapers.

### Background Art

Conventionally, in a manufacturing line of absorbent articles such as disposable diapers, a continuous-sheet-like member that is continuously conveyed is cut off to produce a single-sheet workpiece, and then, this single-sheet workpiece is bonded to another continuous sheet to manufacture a composite body of a continuous-sheet.

### Citation List

### Patent Literature

Patent Literature 1: WO2005-075163
Further prior art in this technical field is disclosed in documents US 2003/051802 A1, JP 2006 340902 A and WO 2014/185242A1.

### Summary of Invention

### Technical Problem

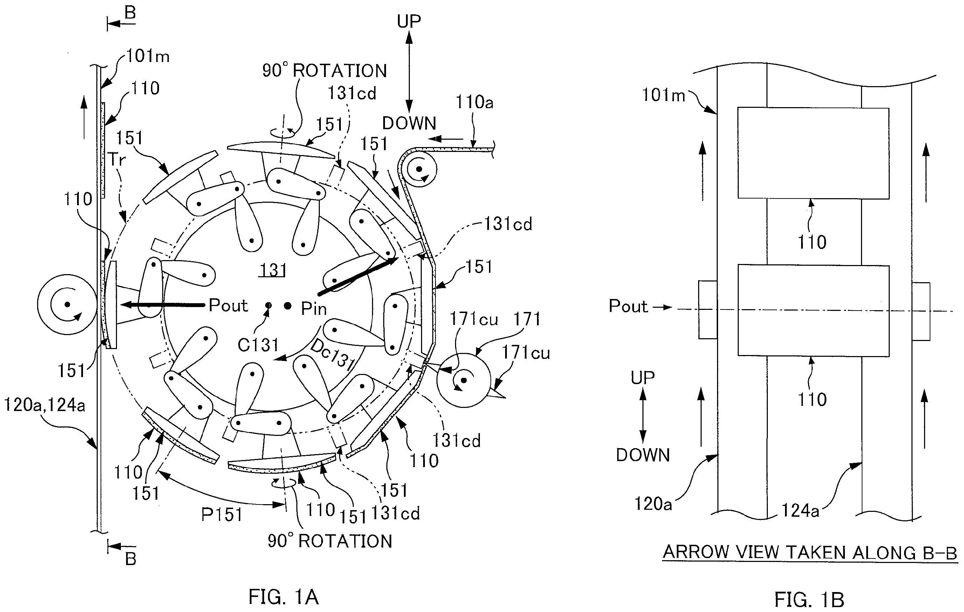

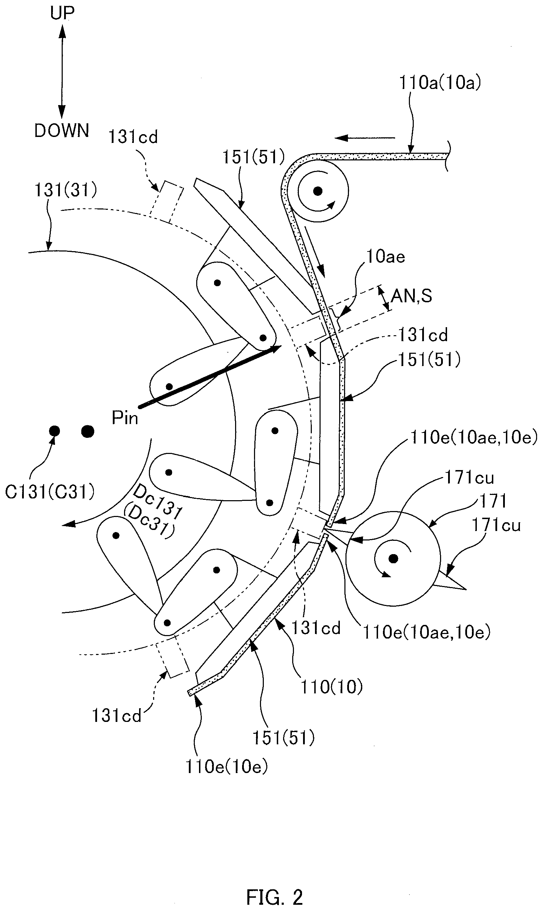

Such a composite body 101m of a continuous-sheet is manufactured, for example, using a rotating drum 131. Fig. 1A is a schematic side view of the rotating drum 131. Fig. 1B is a view along arrows B-B in Fig, 1A. Fig. 2 is a schematic side view illustrating a receiving position Pin and an arrangement position of a cutter roll 171 in an enlarged manner.

The rotating drum 131 includes a plurality of holding members 151, 151, ... that circulates along a circular circulating trajectory Tr centering a predetermined rotation shaft C131. Then, each holding member 151 receives and holds a continuous sheet-like member 110a, which is described above, when each holding member 151 passes through the receiving position Pin on the circulating trajectory Tr. At a downstream position of the receiving position Pin, the cutter roll 171 that rotates at this position is disposed. This cutter roll 171 has an outer peripheral surface on which a cutter blade 171cu as an upper blade 171cu is formed to project. A lower blade 131cd is disposed between the holding members 151, 151. Therefore, when this lower blade 131cd passes through the position of the cutter roll 171 in accordance with circulating movement of the holding member 151, the upper blade 171cu is opposed to the lower blade 131cd by a rotation of the cutter roll 171, and then, these upper blade 171cu and lower blade 131cd cooperatively sandwich the continuous-sheet-like member 110a. Then, this cuts the continuous-sheet-like member 110a at a position between the holding members 151, 151 to produce a single-sheet workpiece 110 on the holding member 151. Thus, the single-sheet workpiece 110 held onto the holding member 151 is conveyed to a delivery position Pout at a further downstream on the circulating trajectory Tr. Until when the single-sheet workpiece 110 reaches this delivery position Pout, in some cases, a direction of the single-sheet workpiece 110 is changed by a 90° rotation of the holding member 151, and a pitch P151 between the holding members 151, 151 adjacent to one another in a circulating direction Dc131 is changed. Then, when the single-sheet workpiece 110 held onto the holding member 151 passes through this delivery position Pout, the single-sheet workpiece 110 is bonded to continuous sheets 120a, 124a, which are described above, that similarly travel through the delivery position Pout, and then delivered to these sheets 120a, 124a. Thus, the above-described composite body 101m of the continuous-sheet is manufactured.

Here, in a case of a mechanically cutter device using the upper blade 171cu and the lower blade 131cd, such as the above-described cutter roll 171, as described above, the lower blade 131cd is disposed between the holding members 151, 151 adjacent to one another in the circulating direction Dc131. However, as illustrated in Fig. 2, this cannot make a clearance S between these holding members 151, 151 smaller than a thickness of the lower blade 131cd. As a result, at the receiving position Pin, a region AN that cannot hold the continuous-sheet-like member 110a is widely generated between these holding members 151, 151. Accordingly, the holding member 151 cannot fixedly hold end portions 110e, 110e of the single-sheet workpiece 110. As a result, this possibly generates unintended deformation, for example, that these end portions 110e get wrinkles and are turned up. Then, if the single-sheet workpiece 110 is bonded to the above-described continuous sheets 120a, 124a in such a deformation state, manufacturing quality of the manufactured composite body 101m of the continuous-sheet is reduced.

The present invention has been made in consideration of the conventional problems as described above. An object of the present invention is to prevent reduction of manufacturing quality of a composite body of a continuous-sheet associated with an absorbent article.

### Solution to Problem

A primal invention to achieve the above-mentioned object is a method for manufacturing a composite body of a continuous sheet associated with an absorbent article by producing a single-sheet workpiece from a continuous-sheet-like member that is continuously conveyed and by bonding the single-sheet workpiece to a continuous sheet, the method including:
receiving and holding the continuous-sheet-like member with a plurality of holding members when each of the plurality of holding members that circulates along a circulating trajectory and is aligned in a circulating direction passes through a receiving position on the circulating trajectory;
producing the single-sheet workpiece by irradiating the continuous-sheet-like member with laser beam while the holding members hold the continuous-sheet-like member, so as to cut off a part held onto the holding members in the continuous-sheet-like member; and
bonding and delivering the single-sheet workpiece to the continuous sheet when the single-sheet workpiece held onto the holding member passes through a delivery position on the circulating trajectory.

Further, a primal invention to achieve the object is an apparatus for manufacturing a composite body of a continuous sheet associated with an absorbent article by producing a single-sheet workpiece from a continuous-sheet-like member that is continuously conveyed and by bonding the single-sheet workpiece to a continuous sheet, the apparatus including:
a plurality of holding members that circulates along a circulating trajectory and is aligned in a circulating direction, each of the plurality of holding members receiving and holding the continuous-sheet-like member when passing through a receiving position on the circulating trajectory;
a laser cutter that cuts off a part held onto the holding members in the continuous-sheet-like member to produce the single-sheet workpiece by irradiating the continuous-sheet-like member with laser beam; and
a bonding device that bonds the single-sheet workpiece to the continuous sheet when the single-sheet workpiece held onto the holding member passes through a delivery position on the circulating trajectory.
Other features of the present invention will become apparent from the description in this specification and the attached drawings.

### Advantageous Effects of Invention

The present invention can prevent the reduction of the manufacturing quality of the composite body of the continuous-sheet associated with the absorbent article. Brief Description of Drawings

[Fig. 1] Fig. 1A is a schematic side view of a rotating drum 131 for describing a conventional problem, and Fig. 1B is a view along arrows B-B in Fig. 1A.
[Fig. 2] Fig. 2 is a schematic side view illustrating a receiving position Pin and an arrangement position of a cutter roll 171 in the above-described rotating drum 131 in an enlarged manner.
[Fig. 3] Fig. 3A is a schematic plan view of a developed state of a diaper 1 that is manufactured using a manufacturing apparatus 30 of the present embodiment, Fig. 3B is a cross-sectional view taken along B-B in Fig. 3A, and Fig. 3C is a schematic perspective view of the diaper 1 when it is worn.
[Fig. 4] Fig. 4A is a schematic side view of the manufacturing apparatus 30 in the preset embodiment, Fig. 4B is a view along arrows B-B in Fig. 4A, and Fig. 4C is a view along arrows C-C in Fig. 4A.
[Fig. 5] Fig. 5A is a schematic plan view of a holding pad 51 viewed from a holding surface 51s, Fig. 5B is a view along arrows B-B in Fig. 5A, and Fig. 5C is a view along arrows C-C in Fig. 5A.
[Fig. 6] Fig. 6 is a schematic side view illustrating a receiving position Pin and an arrangement position of a laser cutter 71 in the above-described manufacturing apparatus 30 in an enlarged manner.

### Description of Embodiment

At least the following matters will be made clear by the description in the present specification and the accompanying drawings.

A method for manufacturing a composite body of a continuous sheet associated with an absorbent article by producing a single-sheet workpiece from a continuous-sheet-like member that is continuously conveyed and by bonding the single-sheet workpiece to a continuous sheet, the method according to the invention being defined by the combination of features according to claim 1 and including:
receiving and holding the continuous-sheet-like member with a plurality of holding members when each of the plurality of holding members that circulates along a circulating trajectory and is aligned in a circulating direction passes through a receiving position on the circulating trajectory;
producing the single-sheet workpiece by irradiating the continuous-sheet-like member with laser beam while the holding members hold the continuous-sheet-like member, so as to cut off a part held onto the holding members in the continuous-sheet-like member; and
bonding and delivering the single-sheet workpiece to the continuous sheet when the single-sheet workpiece held onto the holding member passes through a delivery position on the circulating trajectory.

With the method for manufacturing a composite body of a continuous sheet associated with an absorbent article, the single-sheet workpiece is produced by irradiating the continuous sheet with the laser beam so as to be cut off from the continuous-sheet-like member. Accordingly, the lower blade, which is required to be disposed at a position at the proximity of the holding member in the case of the mechanically cutter device that cuts with the upper blade and the lower blade, can be omitted. Then, this can reduce the clearance between the holding members adjacent to one another in the circulating direction of the circulating trajectory, at the above-described receiving position. As a result, these holding members can receive the continuous-sheet-like member in a state where the interval between these holding members is narrowed so as to mostly contact with one another in the circulating direction. That is, the region that is possibly generated between these holding members and cannot hold the continuous-sheet-like member can be reduced. As a result, the holding member can hold the single-sheet workpiece up to almost both its end portions. Then, this can bond the single-sheet workpiece to the above-described continuous sheet while effectively reducing the unintended deformation of the single-sheet workpiece also at the above-mentioned both end portions. From the above results, the reduction of the manufacturing quality of the composite body of the continuous sheet can be prevented.

In the method for a composite body of a continuous sheet associated with an absorbent article, it is preferable that the continuous-sheet-like member is stretchable, and
the holding member receives the continuous-sheet-like member in an extended state in the circulating direction at the receiving position.

With the method for manufacturing a composite body of a continuous sheet associated with an absorbent article, operational advantage of the above-described deformation reduction of the single-sheet workpiece can be considerably enjoyed. That is, if the clearance between the holding members cannot be reduced at the receiving position, the above-described both end portions in the single-sheet workpiece cannot be held by the holding member. Thus, these both end portions contract and deform such as getting wrinkles based on their stretch. However, on this point, with the above-described manufacturing method, the clearance between the holding members can be reduced by the amount corresponding to the above-described omission of the lower blade. This can fixedly hold the single-sheet workpiece up to both ends. Then, as a result, while the contraction deformation of these both end portions is reduced, the single-sheet workpiece can be bonded to the above-described continuous sheet.

In the method for manufacturing a composite body of a continuous sheet associated with an absorbent article,
it is preferable that before the single-sheet workpiece in a state held onto the holding member reaches the delivery position, a direction of the single-sheet workpiece is changed by a rotation of the holding member.

With the method for manufacturing a composite body of a continuous sheet associated with an absorbent article, operational advantage of the above-described deformation reduction of the single-sheet workpiece can be considerably enjoyed. That is, if the clearance between the holding members cannot be reduced at the receiving position, the above-described both end portions in the single-sheet workpiece cannot be held by the holding member, thus possibly causing these both end portions to be turned up and/or replicate by wind pressure caused by the rotation of the holding member that may further acts in addition to wind pressure caused by the circulating movement. However, on this point, with the above-described manufacturing method, the clearance between the holding members can be reduced by the amount corresponding to the above-described omission of the lower blade. This can fixedly hold the single-sheet workpiece up to both end portions. Then, as a result, while the turn-up and the replication of these both end portions are reduced, the single-sheet workpiece can be bonded to the above-described continuous sheet.

In the method according to the invention for manufacturing a composite body of a continuous sheet associated with an absorbent article further includes that,
when a boundary position between the holding members adjacent to one another in the circulating direction passes through the receiving position, a clearance between the holding members has been reduced to 3 mm or less.

With the method for manufacturing a composite body of a continuous sheet associated with an absorbent article, when the boundary position between the holding members adjacent to one another in the circulating direction passes through the receiving position, these holding members are in a state where the clearance between the holding members one another has been reduced to 3 mm or less. Accordingly, the region that cannot hold the continuous-sheet-like member can be almost eliminated. The region is possibly generated between these holding members. Thereby, the holding member can hold the single-sheet workpiece up to both end portions. As a result, while the unintended deformation of the single-sheet workpiece is surely reduced also at the above-described both end portions, the single-sheet workpiece can be bonded to the above-described continuous sheet.

In the method for manufacturing a composite body of a continuous sheet associated with an absorbent article,
it is preferable that between the delivery position and the receiving position, a pitch between the holding members adjacent to one another in the circulating direction is changed to reduce the clearance between the holding members to 3 mm or less.

With the method for manufacturing a composite body of a continuous sheet associated with an absorbent article, when the above-described boundary position passes through the receiving position, the state where the clearance between the holding members adjacent to one another in the circulating direction has been reduced to 3 mm or less can be ensured.

In the method for manufacturing a composite body of a continuous sheet associated with an absorbent article,
it is preferable that a state where the clearance between the holding members has been reduced to 3 mm or less is at least maintained while the continuous-sheet-like member is irradiated with the laser beam, and
a part corresponding to the boundary position in the continuous-sheet-like member is irradiated with the laser beam to cut off the continuous-sheet-like member.

With the method for manufacturing a composite body of a continuous sheet associated with an absorbent article, the continuous-sheet-like member is cut off in the state where the clearance between the above-described holding members has been reduced to 3 mm or less. Accordingly, the holding member can surely hold the single-sheet workpiece, which is produced by this cut, up to its both end portions.

In the method for manufacturing a composite body of a continuous sheet associated with an absorbent article,
it is preferable that the holding member holds the continuous-sheet-like member on a holding surface that faces outside of the circulating trajectory, and
the continuous-sheet-like member is irradiated with the laser beam from a laser cutter disposed outside the circulating trajectory.

With the method for manufacturing a composite body of a continuous sheet associated with an absorbent article, while the continuous-sheet-like member is irradiated with the laser beam, the holding member can be promptly avoided from becoming a hindrance. That is, the above-described continuous-sheet-like member is positioned outside the circulating trajectory with respect to the holding member. Here, the laser cutter is also disposed outside the circulating trajectory. Accordingly, the laser cutter can irradiate with the laser beam from the outside of the continuous sheet. As a result, in this irradiation, the holding member can be promptly avoided from becoming the hindrance.

In the method for manufacturing a composite body of a continuous sheet associated with an absorbent article,
it is preferable that an irradiation target part in the continuous-sheet-like member is irradiated with the laser beam,
the irradiation target part is positioned between the holding members adjacent to one another in the circulating direction, and
at least both end portions in the circulating direction in the holding member when being irradiated with the laser beam include heat-resistant portions.

With the method for manufacturing a composite body of a continuous sheet associated with an absorbent article, the heat-resistant portions are provided at the above-described both end portions in the holding member. Accordingly, even if by any chance the above-described both end portions of the holding member are irradiated with a part of the laser beam that cuts off the continuous-sheet-like member, thermal damage at both end portions caused by this can be effectively reduced.

Further, an apparatus for manufacturing a composite body of a continuous sheet associated with an absorbent article by producing a single-sheet workpiece from a continuous-sheet-like member that is continuously conveyed and by bonding the single-sheet workpiece to a continuous sheet, the apparatus according to the invention being defined by the combination of features according to claim 8 and including:
a plurality of holding members that circulates along a circulating trajectory and is aligned in a circulating direction, each of the plurality of holding members receiving and holding the continuous-sheet-like member when passing through a receiving position on the circulating trajectory;
a laser cutter that cuts off a part held onto the holding members in the continuous-sheet-like member to produce the single-sheet workpiece by irradiating the continuous-sheet-like member with laser beam; and
a bonding device that bonds the single-sheet workpiece to the continuous sheet when the single-sheet workpiece held onto the holding member passes through a delivery position on the circulating trajectory.

With the apparatus for manufacturing a composite body of a continuous sheet associated with the absorbent article, operational advantages similar to those in the case of the above-described manufacturing method can be provided.

### ===Present Embodiment===

A method for manufacturing a composite body 1m of a continuous-sheet of the present embodiment and a manufacturing apparatus 30 are used, for example, in a manufacturing line of a disposable diaper 1.

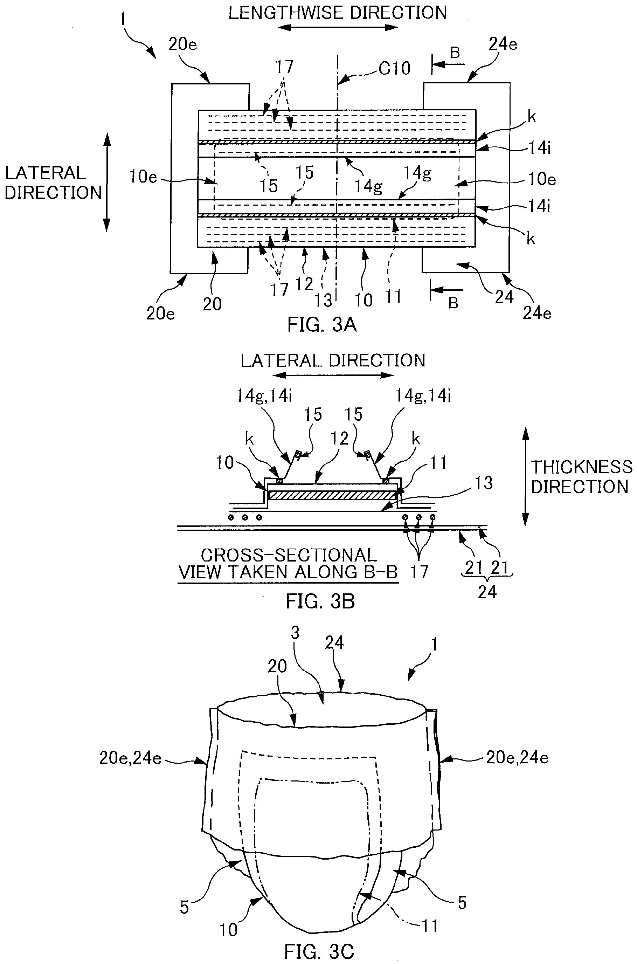

Fig. 3A to Fig. 3C are explanatory views of the disposable diaper 1. Fig. 3A is a schematic plan view of the diaper 1 in a developed state, Fig. 3B is a cross-sectional view taken along B-B in Fig. 3A, and Fig. 3C is a schematic perspective view of the diaper 1 when it is worn.

This diaper 1, in the developed state in Fig. 3A, has a lengthwise direction, a lateral direction, and a thickness direction as three directions that are mutually orthogonal. This diaper 1 is what is called a three-piece type diaper, thus including a front band member 20, a back band member 24, and an absorbent main body 10. The front band member 20 covers a front portion of a wearer. The back band member 24 covers a back portion of the wearer. The absorbent main body 10 is placed on a crotch of the wearer to absorb bodily fluid such as urine. Then, in this developed state, in a state where the front band member 20 and the back band member 24 are mutually aligned in parallel along the lateral direction with a space in the lengthwise direction between them, the absorbent main body 10 is bridged between the front band member 20 and the back band member 24, and both end portions 10e, 10e in the lengthwise direction of this absorbent main body 10 are bonded and fixed to nearest band members 20, 24, thus forming an appearance shape of this diaper 1 into an approximately H shape in a plan view.

Here, from this developed state, the diaper 1 is folded in two taking an approximately center portion C10 in the lengthwise direction of the absorbent main body 10 as a folding position, and the band members 20, 24 opposed to one another in this state folded in two are coupled to one another, for example, by being welded at sites 20e, 24e that should abut on flanks of the wearer. Then, these band members 20, 24 are coupled to one another in an annular shape. This results in the diaper 1 in a wearing state where a waist opening 3 and a pair of leg openings 5, 5 are formed as illustrated in Fig. 3C.

As illustrated in Fig. 3A and Fig. 3B, the absorbent main body 10 includes an absorbent core 11, a top sheet 12, and a back sheet 13. The absorbent core 11 is obtained by forming liquid absorbent material such as pulp fiber and superabsorbent polymer (what is called SAP) into a predetermined shape such as an approximately rectangular shape in a plan view. The top sheet 12 covers the absorbent core 11 from a skin side of the wearer. The back sheet 13 covers the absorbent core 11 from a non skin side. The top sheet 12 is, for example, a fluid-permeable nonwoven fabric having a plane size larger than that of the absorbent core 11. The back sheet 13 is, for example, a fluid-impermeable sheet also having a plane size larger than that of the absorbent core 11. As its example, a fluid-impermeable leak-proof sheet such as polyethylene and a laminated sheet obtained by sticking this leak-proof sheet and an exterior sheet such as a nonwoven fabric (not illustrated) together are included. Then, in a state where these top sheet 12 and back sheet 13 sandwich the absorbent core 11, for example, at parts that projects outside from four sides of the absorbent core 11, the top sheet 12 and the back sheet 13 are bonded to one another in a frame shape. This forms the absorbent main body 10. The absorbent core 11 may be coated with a fluid-permeable sheet (not illustrated) such as tissue paper.

As illustrated in Fig. 3B, elastic members 17 such as elastic strings along the lengthwise direction are secured to parts that project outside in the lateral direction with respect to the absorbent core 11 in the absorbent main body 10, for example, by a hot-melt adhesive in a stretched state in this lengthwise direction. Then, this adds stretch to these parts. Here, these parts are parts that will be the leg openings 5 of the diaper 1. Accordingly, if the stretched state of these parts is released, these parts contract in the lengthwise direction to form a plurality of pleats. Thus, these pleats become leg gathers of the respective leg openings 5, 5 of the diaper 1.

Further, in some cases, as this example, respective end portions in the lateral direction at the top sheet 12 may include barrier cuffs 14g, 14g that stand from a skin side surface of this sheet 12 to prevent side leakage. The respective barrier cuffs 14g are formed by respective barrier cuff sheets 14 fixed to the respective end portions in the lateral direction of the top sheet 12 via fixed portions k. That is, an elastic member 15 such as an elastic string is fixed in the stretched state in the lengthwise direction to an inside part 14i in the lateral direction with respect to the above-described fixed portion k in this sheet 14. Then, when this stretched state is released, this inside part 14i contracts in the lengthwise direction, and thus the inside part 14i stands while forming a plurality of pleats. As a result, this inside part 14i functions as the barrier cuff 14g. The above-described fixed portion k is formed, for example, by a hot-melt adhesive.

The front band member 20 and the back band member 24 are both made of a soft sheet material such as nonwoven fabric. Here, as illustrated in Fig. 3B, the respective band members 20, 24 are formed by stacking nonwoven fabrics 21, 21 together. The respective band members 20, 24 are bonded to the respective end portions 10e, 10e in the lengthwise direction at the absorbent main body 10 respectively. Elastic members (not illustrated) such as elastic strings are fixed to the respective band members 20, 24 in extended states in the lateral direction along this lateral direction. This adds elasticity in the lateral direction to these band members 20, 24.

Such diaper 1 is manufactured in the manufacturing line. In this line, the intermediate product 1m of the diaper 1 is conveyed along a predetermined direction of conveyance. Then, during this conveyance, various kinds of processes are performed on this intermediate product 1m. Each time the respective processes are performed, a form of the intermediate product 1m is sequentially changed, and finally the diaper 1 as in Fig. 3C is completed. The manufacturing method and the manufacturing apparatus 30 of the present embodiment undertake one of the processes.

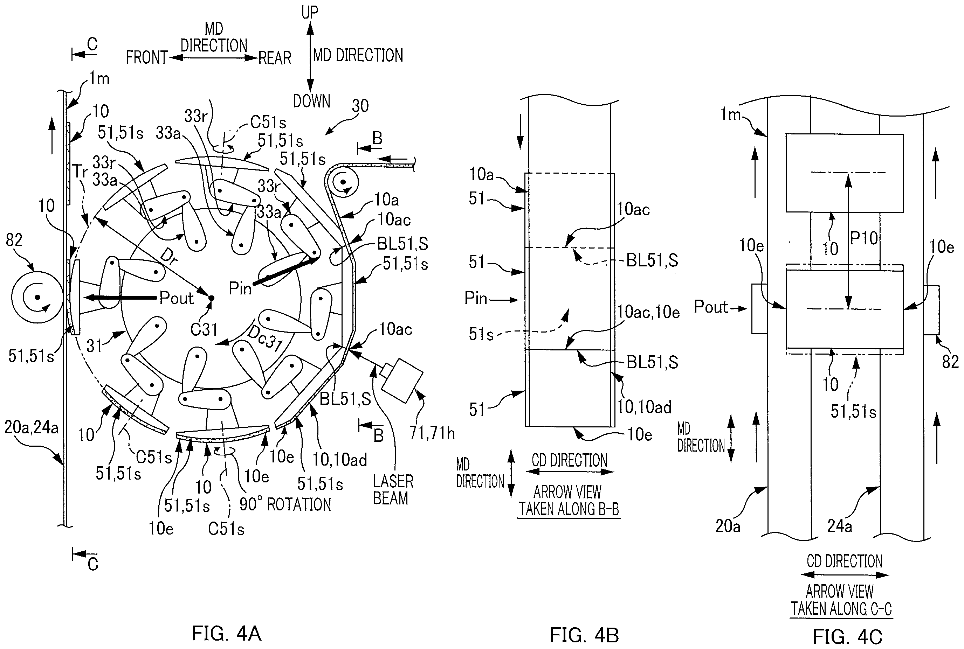

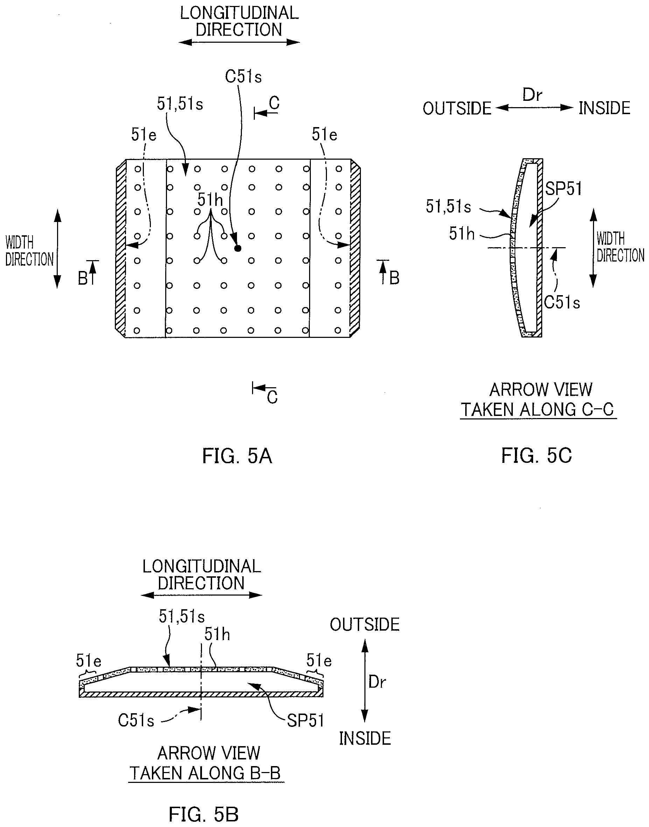

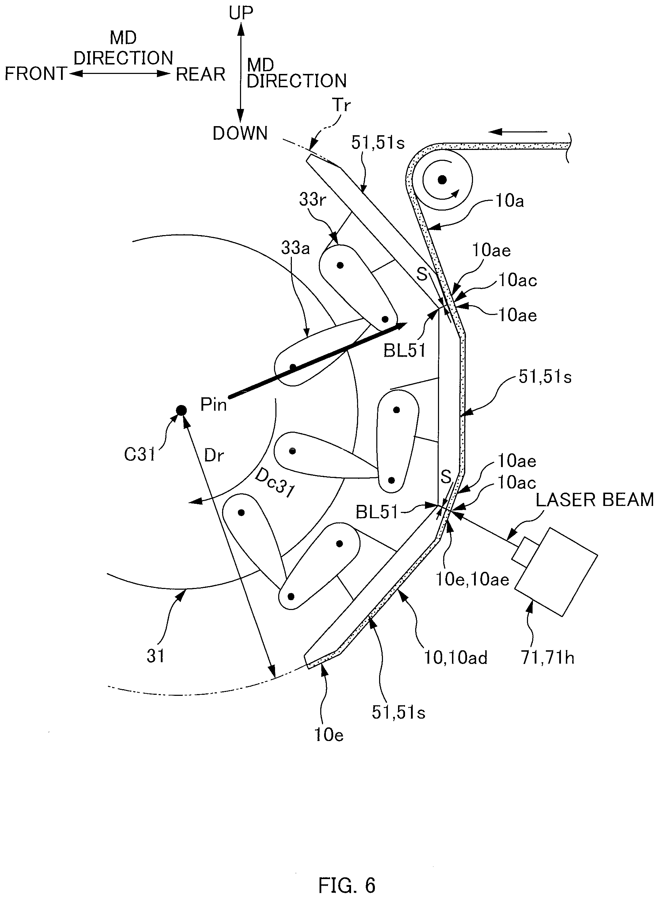

Fig. 4A is a schematic side view of the manufacturing apparatus 30 of the present embodiment. Fig. 4B is a view along arrows B-B in Fig. 4A. Fig. 4C is a view along arrows C-C in Fig. 4A. Fig. 5A is a schematic plan view of a holding pad 51 viewed from a holding surface 51s. Fig. 5B is a view along arrows B-B in Fig. 5A. Fig. 5C is a view along arrows C-C in Fig. 5A. Further, Fig. 6 is a schematic side view illustrating a receiving position Pin and an arrangement position of the laser cutter 71 in this manufacturing apparatus 30 in an enlarged manner.

In the following, a width direction of the manufacturing line is also referred to as a "CD direction," and a direction perpendicular to this CD direction is also referred to as a "MD direction." That is, the MD direction is referred to as any direction in a planar surface perpendicular to the CD direction. Then, the intermediate product 1m of the diaper 1 is conveyed taking the MD direction as the direction of conveyance. In the following, among two directions that are mutually orthogonal in the MD direction, a vertical direction is also referred to as an "up and down direction," and a horizontal direction is also referred to as a "front-rear direction."

As illustrated in Fig. 4A, in this manufacturing apparatus 30, the following process is performed. A continuous body 10a of an absorbent main body (equivalent to the continuous-sheet-like member) formed by connecting a plurality of absorbent main bodies 10, 10, ... in the lengthwise direction is cut off by the laser cutter 71 to produce a single-sheet absorbent main body 10 (equivalent to the single-sheet workpiece), and the produced absorbent main body 10 is extended across and bonded to a pair of band-member continuous bodies 20a, 24a (equivalent to the continuous sheet). Thereby, an approximately ladder-type member 1m (equivalent to the composite body of the continuous-sheet) as in Fig. 4C is manufactured as the intermediate product 1m of the diaper 1.

The following describes the above-described process undertook by this manufacturing apparatus 30.

First, as illustrated in Fig. 4C, the pair of band members 20, 24 at the time being supplied to this manufacturing apparatus 30 are conveyed, in forms of the continuous bodies 20a, 24a along the MD direction, along a continuous direction of these continuous bodies 20a, 24a, and in a state aligned in parallel with an interval between one another in the CD direction.

As illustrated in Fig. 4B, the absorbent main body 10 is also conveyed, in a form of a continuous body 10a that is continuous in the MD direction, along a continuous direction of this continuous body 10a. That is, the top sheet 12, the back sheet 13, and the pair of barrier cuff sheets 14, 14 that constitute the absorbent main body 10 are each in a state of a continuous sheet that is continuous in the MD direction. Then, the absorbent core 11 interposed between the respective continuous sheets of these top sheet 12 and back sheet 13 is in a state aligned with an interval between the absorbent cores 11 adjacent to one another in the MD direction. Then, these respective continuous sheets are in a state mutually integrally bonded to the continuous sheet or the absorbent core 11 that are mutually adjacent in the thickness direction by an adhesion or similar material.

Meanwhile, as illustrated in Fig. 4A, the manufacturing apparatus 30 includes a rotating drum 31. The rotating drum 31 is driven to rotate around a rotation shaft C31 along the CD direction. The rotating drum 31 has an outer periphery portion on which a plurality of holding pads 51, 51, ... (equivalent to the holding members) are disposed aligned in a direction of rotation Dc31 (equivalent to the circulating direction) of the rotating drum 31. The plurality of holding pads 51, 51, ... obtains rotation force from rotating movement of the rotating drum 31. This circulates the plurality of holding pads 51, 51, ... taking a circulating trajectory Tr having a precise circle shape centering the above-described rotation shaft C31 as one direction (clockwise in the example in Fig. 4A).

Each holding pad 51 has the holding surface 51s that generates absorbing force. This holding surface 51s faces an outside in a rotation radial direction Dr of the circulating trajectory Tr. The above-described continuous body 10a of the absorbent main body is conveyed toward the receiving position Pin set on the circulating trajectory Tr. Accordingly, when the respective holding pads 51, 51, ... pass through this receiving position Pin, the respective holding pads 51, 51, ... receive one surface of the continuous body 10a of the absorbent main body by adsorption of the holding surfaces 51s and hold it on these surfaces 51s.

The laser cutter 71 is disposed at a downstream position of the receiving position Pin on the circulating trajectory Tr. Then, when a boundary position BL51 of the holding pads 51, 51 adjacent to one another in the direction of rotation Dc31 passes through the location of the laser cutter 71, a part 10ac corresponding to the above-described boundary position BL51 in the continuous body 10a of the absorbent main body is irradiated with the laser beam from the laser cutter 71. This cuts off a downstream part 10ad of the boundary position BL51 from the continuous body 10a of the absorbent main body. As a result, a single-sheet absorbent main body 10 is produced on the holding pad 51. Then, this holding pad 51 remains to hold the absorbent main body 10 on the holding surface 51s to move to a further downstream delivery position Pout on the circulating trajectory Tr.

In this process to move to the delivery position Pout, the holding pad 51 rotates at 90° around an approximately plane center C51s of the holding surface 51s. Thus, the absorbent main body 10 on the holding surface 51s also rotates at 90° around the approximately plane center C51s. As a result, the lengthwise direction of the absorbent main body 10 is changed from the MD direction to the CD direction. Thereby, the absorbent main body 10 has a posture configured to be extended across the pair of band-member continuous bodies 20a, 24a.

Similarly, in this process to move to the delivery position Pout, a pitch P51 (not illustrated in any drawings) between the holding pads 51, 51 adjacent to one another is enlarged. Its purpose is to prevent interference of the holding pads 51, 51 one another that possibly occurs when the one holding pad 51 rotates, and to adjust the above-described pitch 51 to a size of a mounting pitch P10 (Fig. 4C) when the absorbent main body 10 is bonded to and mounted on the pair of band-member continuous bodies 20a, 24a at the delivery position Pout, which is described below.

On the other hand, in a process to move from this delivery position Pout to the receiving position Pin after passing through this delivery position Pout, the pitch P51 between the holding pads 51, 51 is reduced to return to the original pitch P51. Simultaneously with this reduction or at a time point before this reduction, the holding pad 51 rotates at 90°. This returns the direction of this pad 51 to the original direction before the holding pad 51 arrives at the receiving position Pin. Incidentally, the former change of this pitch P51 is performed by a repitch mechanism, and the latter rotation is performed, for example, by appropriate driving sources such as motors or cam mechanisms provided for each holding pad 51. The repitch mechanism will be described later.

As illustrated in Fig. 4C, at the delivery position Pout, a conveying roller 82 (equivalent to the bonding device) that guides travel of the pair of band-member continuous bodies 20a, 24a is disposed. Then, when the holding pad 51 passes through this delivery position Pout, the conveying roller 82 presses the pair of band-member continuous bodies 20a, 24a to the absorbent main body 10 held onto the holding pad 51. This bonds the respective end portions 10e, 10e in the CD direction of the absorbent main body 10 to the continuous bodies 20a, 24a of the pair of band members 20, 24a respectively. Thereby, the absorbent main body 10 is delivered to the pair of band-member continuous bodies 20a, 24a from the holding pad 51 to produce the approximately ladder-type intermediate product 1m as in Fig. 4C. That is, the approximately ladder-type intermediate product 1m obtained by bonding the plurality of absorbent main bodies 10, 10, ... on the pair of band-member continuous bodies 20a, 24a at a predetermined pitch in the MD direction is produced.

As described above, the process performed by this manufacturing apparatus 30 has been described. The following describes the above-described process in more detail with the description of respective configurations 31, 71 included in this manufacturing apparatus 30 and the description of distinctive matters of the present embodiment.

The rotating drum 31 is driven to rotate around the rotation shaft C31 along the CD direction taking a servo motor or similar motor (not illustrated) as the driving source. As illustrated in Fig. 4A, at the outer periphery portion of the rotating drum 31, a plurality of arm members 33a, 33a, ... are supported swingably around a predetermined fulcrum at intervals of a predetermined angle in the direction of rotation Dc31 of the rotating drum 31. Then, each arm member 33a has a swing end coupled to a corresponding holding pad 51 via an appropriate link member 33r. Each holding pad 51 is guided to circulate along the above-described circulating trajectory Tr having the precise circle shape by an appropriate guiding member (not illustrated) such as a guiding rail having a precise circle shape fixed to a ground or the like. Accordingly, when the rotating drum 31 performs the rotating movement in one direction, the respective holding pads 51, 51, ... obtain rotation force from this rotating movement to circulate along the circulating trajectory Tr in one direction (clockwise in the example in Fig. 4A) in conjunction with the rotating drum 31.

As illustrated in Fig. 5A to Fig. 5C, the holding surface 51s of the holding pad 51 has a longitudinal direction and a width direction corresponding to a planar shape of the absorbent main body 10 in order to ensure holding of the absorbent main body 10 from end to end. Then, in order to smoothly receive the continuous body 10a of the absorbent-main-body at the receiving position Pin, a shape when the holding pad 51 is viewed from the width direction is, for example, as illustrated in Fig. 5B, an approximately equilateral trapezoidal shape whose center portion in the longitudinal direction projects to the outside of the rotation radial direction Dr compared with both end portions. In order to smoothly deliver the absorbent main body 10 at the delivery position Pout, a shape when the holding pad 51 is viewed from the longitudinal direction is, for example, as illustrated in Fig. 5C, an approximately arc shape whose center portion in the width direction projects to the outside of the rotation radial direction Dr compared with both end portions. Further, the rotating drum 31 has the repitch mechanism that changes the pitch P51 between the holding pads 51, 51 adjacent to one another in the direction of rotation Dc31.

Then, as illustrated in Fig. 6, when the boundary position BL51 between the holding pads 51, 51 adjacent to one another in the direction of rotation Dc31 passes through the receiving position Pin, the holding pad 51 causes the longitudinal direction of the holding surface 51s to be along the circulating trajectory Tr to pass through this receiving position Pin in a state where a clearance S between the holding pads 51, 51 has been reduced to 3 mm or less. Thus, the holding pad 51 absorbs and holds the continuous body 10a of the absorbent-main-body on the holding surface 51s.

That is, in this embodiment, since the continuous body 10a of the absorbent main body is cut off by the laser cutter 71, the lower blade is not disposed between the holding pads 51, 51. As a result, as described above, the clearance S between the holding pads 51, 51 can be reduced to 3 mm or less. Then, this can almost eliminate the region AN (Fig. 2) that cannot hold the continuous body 10a of the absorbent main body and that possibly occurs between these holding pads 51, 51. As a result, the holding pad 51 can fixedly hold the absorbent main body 10, which will be produced later by being cut off from the continuous body 10a of the absorbent main body by the laser cutter 71, up to both end portions 10e, 10e in the direction of rotation Dc31 of the holding pad 51. This can surely reduce the deformation such as unintended turning up and replicating of the absorbent main body 10 even at these both end portions 10e, 10e.

As this example, when the direction of the absorbent main body 10 is changed by the rotation of the holding pad 51 after production of this absorbent main body 10, operational advantage of the above-described deformation reduction of both end portions 10e, 10e can be more effectively enjoyed. That is, during the rotation of the holding pad 51 illustrated in Fig. 4A, wind pressure caused by the rotation of the holding pad 51 acts on the absorbent main body 10 absorbed by and held onto this pad 51, in addition to wind pressure caused by circulating movement in direction of rotation Dc31. As a result, turning up and replicating of both end portions 10e, 10e are possibly further promoted.

However, on this point, in this example, the pad 51 can fixedly hold the absorbent main body 10 up to both end portions 10e, 10e through the reduction of the clearance S between the holding pads 51, 51 as described above. Accordingly, this can surely reduce the deformation such as turning up and replicating of these both end portions 10e, 10e.

Further, as this example, also when a processing material of the manufacturing apparatus 30 is a member being stretchable in the MD direction like the continuous body 10a of the absorbent main body, the above-described operational advantage of deformation reduction can be more effectively enjoyed. That is, the continuous body 10a of the absorbent main body is stretchable in the MD direction based on the elastic member 17 of the leg gather and the elastic member 15 of the barrier cuff 14g disposed on the continuous body 10a of the absorbent main body itself. Then, at the receiving position Pin, these elastic members 15, 17 are in extended states in the MD direction. Therefore, as in Fig. 2, if a large clearance S is formed between the holding pads 51, 51 at the receiving position Pin, as described above, this clearance S causes the generation of the region AN that cannot hold the continuous body 10a of the absorbent main body. Thereby, parts 10ae corresponding to these regions AN in this continuous body 10a, that is, the parts 10ae corresponding to both end portions 10e, 10e of the absorbent main body 10 contract and deform in the MD direction to get wrinkles in accordance with the cut of the continuous body 10a of the absorbent main body. As a result, the absorbent main body 10 having wrinkles at both end portions 10e, 10e is bonded to the band-member continuous bodies 20a, 24a.

However, on this point, in this example, as in Fig. 6, the clearance S between the holding pads 51, 51 has been reduced at the receiving position Pin. Accordingly, the holding pad 51 can hold the continuous body 10a of the absorbent main body almost over the boundary position BL51 between the holding pads 51, 51. This can fixedly hold the absorbent main body 10, which is produced on the holding pad 51 after being cut off at this boundary position BL51, up to both end portions 10e, 10e. Then, as a result, contraction deformation of these both end portions 10e, 10e can be also surely reduced.

Now, the above-described repitch mechanism is achieved, for example, using a cam member. That is, on the cam member (not illustrated) fixed to the ground side, for example, a ring groove is formed as a face cam. Engaging elements such as cam followers (not illustrated) to be engaged with this ring groove are disposed on the respective arm members 33a, 33a, ... of Fig. 4A. Then, a cam curve having a shape of the ring groove is configured to a shape corresponding to moving patterns of enlargement and reduction of the above-described pitch P51. Accordingly, when the rotating drum 31 performs the rotating movement, the arm member 33a performs swing movement corresponding to respective rotation positions in the direction of rotation Dc31 of the rotating drum 31 by the engagement of the ring groove as the face cam with the engaging element. This results in achievement of the change of the pitch P51 between the holding pads 51, 51 adjacent to one another.

However, the achievement of the repitch mechanism is not limited to the cam member in any way. For example, the above-described repitch mechanism may be achieved by disposing a guiding member that guides the respective holding pads 51, 51, ... relatively movably in a predetermined range in the direction of rotation Dc31 with respect to the rotating drum 31 and by disposing servo motors for driving of the relative movement in the above-described predetermined range on the respective holding pads 51, 51, ....

The above-described generation of the absorbing force of the holding surface 51s can be achieved, for example, by intake action from a plurality of intake holes 51h, 51h, ... disposed over an approximately whole surface of the holding surface 51s as in Fig. 5A to Fig. 5C. Incidentally, this intake action can be achieved, for example, by compartmentally forming a pressure chamber SP51 communicated with the intake holes 51h, 51h, ... inside the holding pad 51 and by coupling the above-described pressure chamber SP51 to a negative-pressure source (not illustrated) such as a blower or a compressor via an appropriate pipe member (not illustrated). However, the location of the intake holes 51h is not limited to the approximately whole surface of the holding surface 51s as described above in any way. For example, the intake holes 51h may be disposed only at respective edge portions of the four sides of the holding surface 51s and their proximity parts without disposing the intake holes 51h at a proximity part of the approximately plane center C51s of the holding surface 51.

Meanwhile, as illustrated in Fig. 6, the state where the clearance S has been reduced is maintained until when the boundary position BL51 between the holding pads 51, 51 passes through the location of the laser cutter 71. Further, this reduced state is at least maintained while the continuous body 10a of the absorbent main body is irradiated with laser beam. Accordingly, in this state where the clearance S has been reduced, the laser cutter 71 irradiates the irradiation target part 10ac corresponding to the above-described boundary position BL51 in the continuous body 10a of the absorbent main body with laser beam. This cuts off the downstream part 10ad from the continuous body 10a of the absorbent main body at this part 10ac. Then, as a result, onto the holding pad 51 positioned at a downstream side of the above-described holding pads 51, 51, the above-described cut downstream part 10ad is held as the single-sheet absorbent main body 10.

This laser cutter 71, as illustrated in Fig. 4A, includes an irradiation head 71h that can move a spot of the laser beam in the CD direction and the direction of rotation Dc31, and a control unit (not illustrated) that controls the irradiation head 71h. Then, when the boundary position BL51 between the holding pads 51, 51 passes through, in conjunction with this pass, the above-described spot is moved in the CD direction and the direction of rotation Dc31 to cut off the continuous body 10a of the absorbent main body along the CD direction at this boundary position BL51. For this laser cutter 71, one having a well-known configuration is available. As its example, a two-shaft type galvanometer scanner is included. That is, this galvanometer scanner includes a light source of the laser beam, a lens, and a galvanometer mirror that changes an irradiation direction of the laser beam to two directions. This ensures scan of the laser beam in two directions. For the above-described light source, an appropriate light source is available if fusing of a cut target is possible. For example, a diode type may be used, or a CO₂ type may be used.

As illustrated in Fig. 4A, in this example, the irradiation head 71h of the laser cutter 71 is disposed at a position outside the rotation radial direction Dr with respect to the circulating trajectory Tr of the holding pad 51. Accordingly, from the position outside the rotation radial direction Dr with respect to the continuous body 10a of the absorbent main body, the continuous body 10a of the absorbent main body can be irradiated with the laser beam. As a result, in the irradiation of the laser beam, the holding pad 51 is effectively prevented from becoming an obstacle. However, the invention is not limited thereto. That is, if a location space is ensured, the irradiation head 71h may be disposed inside the circulating trajectory Tr.

Preferably, as illustrated in Fig. 5A, heat-resistant portions are disposed on the parts 51e, 51e (hatched parts in Fig. 5A) on which the irradiation target parts 10ac of the continuous body 10a of the absorbent main body abut in the holding pad 51 and their proximity parts. That is, although only the continuous body 10a of the absorbent main body can be irradiated with the laser beam by adjustment of a focus distance of the laser beam, a dimension in the thickness direction possibly varies at the above-described irradiation target parts 10ac. Accordingly, the parts 51e, 51e on which the above-described irradiation target parts 10ac abut in the holding pad 51 is also possibly irradiated with the laser beam through the continuous body 10a of the absorbent main body. Therefore, if the heat-resistant portion is disposed at this part 51e, if by any chance the part 51e is irradiated with the laser beam, thermal damage such as erosion caused by this can be reduced. Incidentally, in this example, both ends 51e, 51e in the holding pad 51 when passing through the laser cutter 71 correspond to the above-described abutted parts 51e. Therefore, the heat-resistant portion is each disposed at these both end portions 51e, 51e. This installation of the heat-resistant portions may be achieved by performing a thermal process such as application of a heat-resistant inorganic-system coating agent on the above-described parts 51e of the holding pad 51, or may be achieved by mounting liner members, for example, made of heat-resistant steel, made of heat-resistant alloy, and made of ceramic instead of the above-described parts 51e.

### ===Other Embodiments===

While the embodiments of the present invention are described above, the embodiments are intended for easy understanding of the present invention and are not in any way to be construed as limiting the present invention. Needless to say, the present invention may be modified and improved without departing from the scope of the invention, and equivalents thereof are also encompassed by the invention. For example, the following modifications are possible.

While the above-described embodiment exemplifies the disposable diaper 1 used by human as the absorbent article, insofar as the absorbent article that absorbs excretory fluid, this should not be construed in a limiting sense by any means. For example, the absorbent article may be a disposable diaper for animal other than the human, such as a pet.

While the above-described embodiment exemplifies the continuous body 10a of the absorbent main body as the continuous-sheet-like member, exemplifies the absorbent main body 10 as the single-sheet workpiece, and exemplifies the pair of band-member continuous bodies 20a, 24a as the continuous sheet since the absorbent article is the three-piece type disposable diaper 1, this should not be construed in a limiting sense by any means. For example, the absorbent article may be a two-piece type disposable diaper. That is, this absorbent article may be a diaper with a type including an exterior sheet having a front portion, a crotch portion, and a back portion as a first component and including the absorbent main body 10 fixed to a skin side surface of this exterior sheet as a second component. Then, also in this case, the continuous body 10a of the absorbent main body and the absorbent main body 10 correspond to "the continuous-sheet-like member" and "the single-sheet workpiece" respectively. However, "the continuous sheet" will correspond to a continuous body of an exterior-sheet (a continuous body obtained by coupling a plurality of the above-described exterior sheets).

While the above-described embodiment performs both movements of the change of the pitch P51 between the holding pads 51, 51 and the rotation of the respective holding pads 51 between the receiving position Pin and the delivery position Pout as illustrated in Fig. 4A, this should not be construed in a limiting sense by any means. That is, only one of both movements may be performed, or both are not necessary to be performed.

While the above-described embodiment exemplifies the conveying roller 82 as the bonding device disposed at the delivery position Pout as illustrated in Fig. 4A, this should not be construed in a limiting sense by any means. For example, a circulating endless belt may be disposed at this position Pout instead of the conveying roller 82. Then, while the travel of the band-member continuous bodies 20a, 24a is guided by an outer peripheral surface of this endless belt, these continuous bodies 20a, 24a may be pressed to a side of the absorbent main body 10 of the holding pad 51.

While in the above-described embodiment, the shape of the cutting line when the continuous body 10a of the absorbent main body is cut is a straight line along the CD direction, this should not be construed in a limiting sense by any means. That is, the shape may be a curved line such as a circular arc, or may be a complicated shape with a combination of a curved line and a straight line.

### Reference Signs List

- 1: disposable diaper (absorbent article)
- 1m: intermediate product (composite body of continuous sheet)
- 3: waist opening
- 5: leg opening
- 10: absorbent main body (single-sheet workpiece)
- 10a: continuous body of absorbent main body (continuous-sheet-like member)
- 10ac: part (irradiation target part)
- 10ad: downstream part
- 10ae: part
- 10e: end portion
- 11: absorbent core
- 12: top sheet
- 13: back sheet
- 14: barrier cuff sheet
- 14g: barrier cuff
- 14i: part
- 15: elastic member
- 17: elastic member
- 20: front band member (continuous sheet)
- 20a: continuous body of front-band-member (continuous sheet)
- 20e: site
- 21: nonwoven fabric
- 24: back band member
- 24a: continuous body of back band member (continuous sheet)
- 24e: site
- 30: manufacturing apparatus
- 31: rotating drum
- 33a: arm member
- 33r: link member
- 51: holding pad (holding member)
- 51s: holding surface
- 51e: part (both end)
- 51h: intake hole
- 71: laser cutter
- 71h: irradiation head
- 82: conveying roller (bonding device)
- AN: region that cannot hold
- Tr: circulating trajectory
- S: clearance
- ***k***: fixed portion
- C10: approximately center portion
- C31: rotation shaft
- C51s: approximately plane center
- Pin: receiving position
- Pout: delivery position
- BL51: boundary position
- SP51: pressure chamber

## Claims

1. A method for manufacturing a composite body of a continuous sheet associated with an absorbent article by producing a single-sheet workpiece from a continuous-sheet-like member that is continuously conveyed and by bonding the single-sheet workpiece to a continuous sheet, the method comprising:
receiving and holding the continuous-sheet-like member with a plurality of holding members when each of the plurality of holding members that circulates along a circulating trajectory and is aligned in a circulating direction passes through a receiving position on the circulating trajectory;
producing the single-sheet workpiece by irradiating the continuous-sheet-like member with laser beam while the holding members hold the continuous-sheet-like member, so as to cut off a part held onto the holding members in the continuous-sheet-like member; and
bonding and delivering the single-sheet workpiece to the continuous sheet when the single-sheet workpiece held onto the holding member passes through a delivery position on the circulating trajectory, wherein
when a boundary position between the holding members adjacent to one another in the circulating direction passes through the receiving position, a clearance between the holding members has been reduced to 3 mm or less.

2. The method for manufacturing a composite body of a continuous sheet associated with an absorbent article according to claim 1, wherein
the continuous-sheet-like member is stretchable, and
the holding member receives the continuous-sheet-like member in an extended state in the circulating direction at the receiving position.

3. The method for manufacturing a composite body of a continuous sheet associated with an absorbent article according to claim 1 or 2, wherein
before the single-sheet workpiece in a state held onto the holding member reaches the delivery position, a direction of the single-sheet workpiece is changed by a rotation of the holding member.

4. The method for manufacturing a composite body of a continuous sheet associated with an absorbent article according to claim 1, wherein
between the delivery position and the receiving position, a pitch between the holding members adjacent to one another in the circulating direction is changed to reduce the clearance between the holding members to 3 mm or less.

5. The method for manufacturing a composite body of a continuous sheet associated with an absorbent article according to claim 1 or 4, wherein
a state where the clearance between the holding members has been reduced to 3 mm or less is at least maintained while the continuous-sheet-like member is irradiated with the laser beam, and
a part corresponding to the boundary position in the continuous-sheet-like member is irradiated with the laser beam to cut off the continuous-sheet-like member.

6. The method for manufacturing a composite body of a continuous sheet associated with an absorbent article according to any one of claims 1 to 5, wherein
the holding member holds the continuous-sheet-like member on a holding surface that faces outside of the circulating trajectory, and
the continuous-sheet-like member is irradiated with the laser beam from a laser cutter disposed outside the circulating trajectory.

7. The method for manufacturing a composite body of a continuous sheet associated with an absorbent article according to any one of claims 1 to 6, wherein
an irradiation target part in the continuous-sheet-like member is irradiated with the laser beam,
the irradiation target part is positioned between the holding members adjacent to one another in the circulating direction, and
at least both end portions in the circulating direction in the holding member when being irradiated with the laser beam include heat-resistant portions.

8. An apparatus for manufacturing a composite body of a continuous sheet associated with an absorbent article by producing a single-sheet workpiece from a continuous-sheet-like member that is continuously conveyed and by bonding the single-sheet workpiece to a continuous sheet, the apparatus comprising:
a plurality of holding members that circulates along a circulating trajectory and is aligned in a circulating direction, each of the plurality of holding members receiving and holding the continuous-sheet-like member when passing through a receiving position on the circulating trajectory;
a laser cutter that cuts off a part held onto the holding members in the continuous-sheet-like member to produce the single-sheet workpiece by irradiating the continuous-sheet-like member with laser beam; and
a bonding device that bonds the single-sheet workpiece to the continuous sheet when the single-sheet workpiece held onto the holding member passes through a delivery position on the circulating trajectory, wherein the apparatus is operable that when a boundary position between the holding members adjacent to one another in the circulating direction passes through the receiving position, a clearance between the holding members has been reduced to 3 mm or less.

## Patentansprüche

1. Ein Verfahren zur Herstellung eines Verbundkörpers einer Endloslage, die mit einem absorbierenden Artikel durch Herstellung eines einlagigen Werkstücks aus einem endloslagen-artigen Element, das kontinuierlich befördert wird, und durch Binden des einlagigen Werkstücks zu einer Endloslage verbunden ist, das Verfahren umfassend:
Aufnehmen und Halten des endloslagen-artigen Elements mit einer Vielzahl von Halteelementen wenn jedes der Vielzahl von Halteelementen, das entlang einer zirkulierenden Bewegungsbahn zirkuliert und in einer zirkulierenden Richtung aneinandergereiht ist, eine Aufnahmeposition auf der zirkulierenden Bewegungsbahn durchläuft;
Erzeugen des einlagigen Werkstücks durch Bestrahlen des endloslagen-artigen Elements mit Laserstrahlen während die Halteelemente das endloslagen-artige Element halten, um ein Stück, das auf den Halteelementen gehalten wird, in dem endloslagen-artigen Element abzuschneiden; und
Binden und Fördern des einlagigen Werkstücks zu der Endloslage wenn das einlagige Werkstück, das auf dem Halteelement gehalten wird, eine Förderposition auf der zirkulierenden Bewegungsbahn durchläuft, wobei wenn eine Grenzposition zwischen den Halteelementen, die aneinander in der zirkulierenden Richtung angrenzen, die Aufnahmeposition durchläuft, ein Zwischenraum zwischen den Halteelementen auf 3 mm oder weniger reduziert wurde.

2. Das Verfahren zur Herstellung eines Verbundkörpers einer Endloslage, die mit einem absorbierenden Artikel verbunden ist, gemäß Anspruch 1, wobei
das endloslagen-artige Element dehnbar ist, und
das Halteelement das endloslagen-artige Element in einem gedehnten Zustand in der zirkulierenden Richtung an der Aufnahmeposition aufnimmt.

3. Das Verfahren zur Herstellung eines Verbundkörpers einer Endloslage, die mit einem absorbierenden Artikel verbunden ist, gemäß Anspruch 1 oder 2, wobei
bevor das einlagige Werkstück in einem auf dem Halteelement gehaltenen Zustand die Förderposition erreicht, eine Richtung des einlagigen Werkstücks durch eine Rotation des Halteelements geändert wird.

4. Das Verfahren zur Herstellung eines Verbundkörpers einer Endloslage, die mit einem absorbierenden Artikel verbunden ist, gemäß Anspruch 1, wobei
zwischen der Förderposition und der Aufnahmeposition, ein Abstand zwischen den Halteelementen, die in der zirkulierenden Richtung aneinander angrenzen, verändert wird, um den Zwischenraum zwischen den Halteelementen auf 3 mm oder weniger zu reduzieren.

5. Das Verfahren zur Herstellung eines Verbundkörpers einer Endloslage, die mit einem absorbierenden Artikel verbunden ist, gemäß Anspruch 1 oder 4, wobei
ein Zustand, in dem der Zwischenraum zwischen den Halteelementen auf 3 mm oder weniger reduziert wurde, wenigstens währendem das endloslagen-artige Element mit dem Laserstrahl bestrahlt wird, aufrechterhalten wird und
ein Teil, das zu der Grenzposition in dem endloslagen-artigen Element korrespondiert, mit dem Laserstrahl bestrahlt wird, um das endloslagen-artige Element abzuschneiden.

6. Das Verfahren zur Herstellung eines Verbundkörpers einer Endloslage, die mit einem absorbierenden Artikel verbunden ist, gemäß einem der Ansprüche 1 bis 5, wobei
das Halteelement das endloslagen-artige Element auf einer Halteoberfläche, die von der zirkulierenden Bewegungsbahn nach außen gerichtet ist, hält und
das endloslagen-artige Element mit dem Laserstrahl von einem Laserschneider, der außerhalb der zirkulierenden Bewegungsbahn angeordnet ist, bestrahlt wird.

7. Das Verfahren zur Herstellung eines Verbundkörpers einer Endloslage, die mit einem absorbierenden Artikel verbunden ist, gemäß einem der Ansprüche 1 bis 6, wobei
ein Bestrahlungszielteil in dem endloslagen-artigen Element mit dem Laserstrahl bestrahlt wird,
das Bestrahlungszielteil zwischen den Halteelementen, die in der zirkulierenden Richtung aneinander angrenzen, angeordnet ist, und
wenigstens beide Endteile in der zirkulierenden Richtung in dem Halteelement, während sie mit dem Laserstrahl bestrahlt werden, wärmebeständige Teile umfassen.

8. Eine Vorrichtung zur Herstellung eines Verbundkörpers einer Endloslage, die mit einem absorbierenden Artikel durch Herstellung eines einlagigen Werkstücks aus einem endloslagen-artigen Element, das kontinuierlich befördert wird, und durch Binden des einlagigen Werkstücks zu einer Endloslage verbunden ist, die Vorrichtung umfassend:
eine Vielzahl von Halteelementen, die entlang einer zirkulierenden Bewegungsbahn zirkuliert und in einer zirkulierenden Richtung aneinandergereiht ist, jedes der von der Vielzahl von Halteelementen das endloslagen-artige Element beim Durchlaufen einer Aufnahmeposition auf der zirkulierenden Bewegungsbahn aufnehmend und haltend;
ein Laserschneider, der ein Teil, das auf den Halteelementen gehalten wird, in dem endloslagen-artigen Element abschneidet, um das einlagige Werkstück durch Bestrahlung des endloslagen-artigen Elements mit dem Laserstrahl zu erzeugen; und
ein Bindegerät, das das einlagige Werkstück an die Endloslage bindet, wenn das einlagige Werkstück, das auf dem Halteelement gehalten wird, eine Förderposition auf der zirkulierenden Bewegungsbahn durchläuft, wobei die Vorrichtung betriebsbereit ist, dass wenn die Grenzposition zwischen den in der zirkulierenden Richtung aneinander angrenzenden Halteelementen die Aufnahmeposition durchläuft, ein Zwischenraum zwischen den Halteelementen auf 3 mm oder weniger reduziert wurde.

## Revendications

1. Procédé de fabrication d'un corps composite d'une feuille continue associée un article absorbant en produisant une pièce à traiter à feuille unique à partir d'un élément de type stratiforme continu qui est transporté continument et en liant la pièce à traiter à feuille unique à une feuille continue, le procédé comprenant :
la réception et le maintien de l'élément de type stratiforme continu avec une pluralité d'éléments de maintien quand chacun de la pluralité d'éléments de maintien qui circule le long d'une trajectoire de circulation et est aligné dans une direction de circulation passe par une position de réception sur la trajectoire de circulation ;
la production de la pièce à traiter à feuille unique en irradiant l'élément de type stratiforme continu avec un faisceau laser pendant que les éléments de maintien maintiennent l'élément de type stratiforme continu, de façon à couper une partie maintenue sur les éléments de maintien dans l'élément de type stratiforme continu ; et
la liaison et la fourniture de la pièce à traiter à feuille unique à la feuille continue quand la pièce à traiter à feuille unique maintenue sur l'élément de maintien passe par une position de fourniture sur la trajectoire de circulation, dans lequel quand une position limite entre les éléments de maintien adjacents les uns aux autres dans la direction de circulation passe par la position de réception, un dégagement entre les éléments de maintien a été réduit à 3 mm ou moins.

2. Procédé de fabrication d'un corps composite d'une feuille continue associée à un article absorbant selon la revendication 1, dans lequel
l'élément de type stratiforme continu est étirable, et
l'élément de maintien reçoit l'élément de type stratiforme continu dans un état étendu dans la direction de circulation à la position de réception.

3. Procédé de fabrication d'un corps composite d'une feuille continue associée un article absorbant selon la revendication 1 ou 2, dans lequel
avant que la pièce à traiter à feuille unique dans un état maintenue sur l'élément de maintien n'atteigne la position de fourniture, une direction de la pièce à traiter à feuille unique est changée par une rotation de l'élément de maintien.

4. Procédé de fabrication d'un corps composite d'une feuille continue associée à un article absorbant selon la revendication 1, dans lequel
entre la position de fourniture et la position de réception, un pas entre les éléments de maintien adjacents les uns aux autres dans la direction de circulation est changé pour réduire le dégagement entre les éléments de maintien à 3 mm ou moins.

5. Procédé de fabrication d'un corps composite d'une feuille continue associée à un article absorbant selon la revendication 1 ou 4, dans lequel
un état où le dégagement entre les éléments de maintien a été réduit à 3 mm ou moins est au moins maintenu tandis que l'élément de type stratiforme continu est irradié avec le faisceau laser, et
une partie correspondant à la position limite dans l'élément de type stratiforme continu est irradiée avec le faisceau laser pour couper l'élément de type stratiforme continu.

6. Procédé de fabrication d'un corps composite d'une feuille continue associée à un article absorbant selon l'une quelconque des revendications 1 à 5, dans lequel
l'élément de maintien maintient l'élément de type stratiforme continu sur une surface de maintien qui est orientée vers l'extérieur de la trajectoire de circulation, et
l'élément de type stratiforme continu est irradié avec le faisceau laser à partir d'un découpeur au laser disposé à l'extérieur de la trajectoire de circulation.

7. Procédé de fabrication d'un corps composite d'une feuille continue associée à un article absorbant selon l'une quelconque des revendications 1 à 6, dans lequel
une partie cible d'irradiation dans l'élément de type stratiforme continu est irradiée avec le faisceau laser,
la partie cible d'irradiation est positionnée entre les éléments de maintien adjacents les uns aux autres dans la direction de circulation, et
au moins les deux parties d'extrémité dans la direction de circulation dans l'élément de maintien lorsqu'elles sont irradiées avec le faisceau laser incluent des parties résistantes à la chaleur.

8. Appareil de fabrication d'un corps composite d'une feuille continue associée à un article absorbant en produisant une pièce à traiter à feuille unique à partir d'un
élément de type stratiforme continu qui est transporté continument et en liant la pièce à traiter à feuille unique à une feuille continue, l'appareil comprenant :
une pluralité d'éléments de maintien qui circule le long d'une trajectoire de circulation et est alignée dans une direction de circulation, chacun de la pluralité d'éléments de maintien recevant et maintenant l'élément de type stratiforme continu quand il passe par une position de réception sur la trajectoire de circulation ;
un découpeur au laser qui coupe une partie maintenue sur les éléments de maintien dans l'élément de type stratiforme continu pour produire la pièce à traiter à feuille unique en irradiant l'élément de type stratiforme continu avec un faisceau laser ; et
un dispositif de liaison qui lie la pièce à traiter à feuille unique à la feuille continue quand la pièce à traiter à feuille unique maintenue sur l'élément de maintien passe par une position de fourniture sur la trajectoire de circulation, dans lequel l'appareil est utilisable de sorte que lorsqu'une position limite entre les éléments de maintien adjacents les uns aux autres dans la direction de circulation passe par la position de réception, un dégagement entre les éléments de maintien a été réduit à 3 mm ou moins.
